# EUROPEAN PATENT APPLICATION

(11) **EP 0 974 345 A2**
(43) Date of publication of application: **26.01.2000**
(21) Application number: 99305255.4
(22) Date of filing: 02.07.1999
(51) Int. Cl.: A61K 9/48, A61K 9/58, A61K 9/60

(54) **Capsule sealing system**

(30) Priority: 03.07.1998 GB 9814411
(71) Applicant: MW Encap Ltd., Livingston, West Lothian EH54 8SB (GB)
(72) Inventor: Young, Victor Morrison, c/o M W Encap Limited, West Lothian EH5 8SB (GB)
(74) Representative: Kennedy, David Anthony

(57) **Abstract**

A solution for sealing capsules comprises a mixture of the chemical material of the capsule, a solute elevating the thermal gelation temperature, a gelling agent and a hydrating agent. The properties of the solution can be altered by the inclusion of other reagents, for example plasticisers. The solution is produced by mixing a first subordinate solution, which contains the material of the capsule and the solute elevating the thermal gelation temperature, and a second subordinate solution, which contains the gelling agent and the hydrating agent.

## Description

This invention relates to a capsule sealing solution and system especially but not exclusively for use with non-gelatin capsules.

Capsules are one of the major dosage forms for medicinal and health food products. These capsules are made from gelatin, a natural product, derived from animal bone, pigs and cattle being the major source of the bone. However, increasingly problems are being encountered regarding the use of such capsules as a cause of public concern over BSE and also as a result of other issues such as religious beliefs and vegetarianism.

For many years the sealing of liquids and semi-solids into capsules has been done using hard gelatin capsules. Gelatin capsules are sealed by applying a band of hot gelatin solution to bridge the body and cap of a capsule. On drying, a tough leak-proof seal between the two halves of the capsule is formed.

Recent research into encapsulation of liquids and semi-solids has been targeted towards the development of capsules based on the use of hydroxy propyl methyl cellulose (HPMC). Sealing systems for these capsules have been based on the use of HPMC solutions in water and at best have shown limited success.

The best type of solution for sealing a capsule is one based on the material from which the capsule is made. For example, the sealant for gelatin capsules is a gelatin/water mixture that is applied hot and sets (gels) on cooling.

It should therefore be possible to use an HPMC solution to seal a capsule. HPMC solutions do not gel when cold and therefore could be applied cold and subsequently dried. The use of a hot aqueous solution of HPMC is not possible due to the fact that such a solution gels on heating. Gelled solutions are not suitable for use as sealants.

However, on applying the sealant solution, the water of the solution is absorbed by the capsule shell weakening the shell to such an extent that it can be punctured; wet HPMC has little or no strength. The act of closing the cap results in a build up of pressure within the capsule, due to the presence of a gas space. This build up in pressure can cause the contents of the capsule to burst through the wall of a shell weakened by water.

Secondly, between the body and cap of the capsule there exists a space which is filled with the aqueous sealing solution, the solution being drawn into this gap by capillary action. The gas in this gap, therefore, becomes pressurised and seeks a route out of the capsule.

This is normally achieved by forcing a channel through the seal creating a gap in the seal.

It would therefore be desirable to be able to use an HPMC based sealant that will readily gel on application thus slowing absorption of water by the shell and preventing movement of the sealant between the two halves of the capsule by capillary action. The main problems that would require to be overcome as a result of the presence of HPMC include a possible lack of solubility of the gelling agents, gelation on elevation of temperature and achieving gelation of the solution on cooling.

Further requirements of materials to be used in the sealing solution include that they are compatible with the material of the shell, i.e. they can stick to the shell, they are soluble in suitable solvents and that they produce tack free seals. The materials must also be acceptable to the customer and economical.

HPMC based sealing solutions that gel on cooling are not known in the prior art.

It is an object of the present invention to provide a sealing mixture with the necessary properties to form a viable HPMC capsule sealing solution.

According to a first aspect of the present invention there is provided a solution for sealing capsules comprising a mixture of the chemical material of the capsule, a solute elevating the thermal gelation temperature and a gelling agent.

Preferably any suitable capsule material may be used. Typically the material is hydroxy propyl methyl cellulose.

Preferably the grade of viscosity of the hydroxy propyl methyl cellulose is not limited. Typically a low viscosity grade is used.

Preferably any suitable solute elevating the thermal gelation temperature of the chemical material of the capsule may be used. Typically this solute is an alcohol. Typically the alcohol is propan-1-ol or propan-2-ol.

More preferably the proportion of the alcohol in the solution is not limited.

Preferably a plasticiser may be added to modify the properties of the seal. Typically the plasticiser is propylene glycol.

Preferably the gelling agent is comprised of one or more gums. Typically gellan gum is used.

A hydrating agent may also be included to aid the formation of a solution of the gum.

Preferably any suitable hydrating agent may be used. Optionally the hydrating agent may be sodium citrate.

According to a second aspect of the present invention there is provided a process for producing a solution for sealing capsules comprising the mixing of a first and second subordinate solution, wherein the first solution is aqueous and contains the chemical material of the capsule.

Plasticisers also may be added to the first solution.

The first solution may further include a solute.

Preferably the second solution contains a gelling agent and a hydrating agent.

Preferably the subordinate solutions are heated prior to mixing to form the sealing solution. Typically, but not essentially, the temperature of the first solution may be raised to 70°C without gelling.

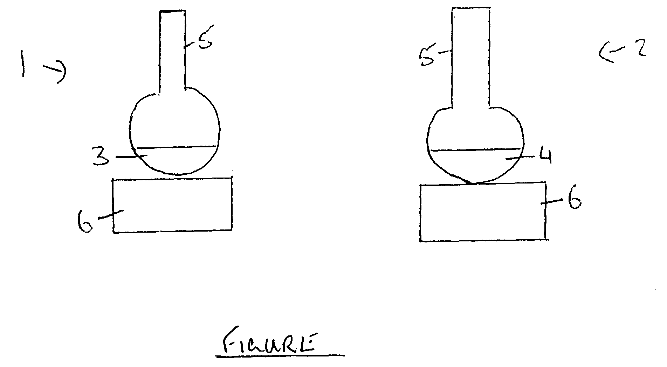

In order to provide a better understanding of the invention, an example of the invention will now be described, with reference to the accompanying Figure which represents the process of making a sealing solution in accordance with the present invention.

The two components containing the material of the capsule and the gelling agent respectively are generally depicted at 1 and 2 each comprising a solution 3 and 4 respectively in a standard flask 5. Both the solutions are heated to a suitable temperature, typically 50°C, on standard stirrer hotplates 6 or by other suitable means. On reaching the desired temperature the solutions are mixed thoroughly, by pouring one solution into the other, and used. The final solution is used to seal capsules using techniques known to persons skilled in the art.

An advantage of the present invention is that there is provided an alternative gelling capsule sealing solution to simple aqueous HPMC based solutions.

A further advantage is that the sealing solution is based on the use of cheap materials. The process for making the solutions is also simple and economical using equipment that is currently in use.

Further modifications and improvements may be added without departing from the scope of the invention herein intended.

## Claims

1. A solution for sealing capsules comprising a mixture of the chemical material of the capsule, a solute elevating the thermal gelation temperature and a gelling agent.

2. A solution as claimed in Claim 1 wherein the chemical material of the capsule is hydroxy propyl methyl cellulose.

3. A solution as claimed in Claims 1 or 2 wherein the grade of viscosity of the hydroxy propyl methyl cellulose is low.

4. A solution as claimed in any of the preceding Claims wherein the solute elevating the thermal gelation temperature of the chemical material of the capsule is an alcohol.

5. A solution as claimed in Claim 4 wherein the proportion of the alcohol in the solution is not limited.

6. A solution as claimed in any of the preceding Claims wherein a plasticiser is added to modify the properties of the seal.

7. A solution as claimed in Claim 6 wherein the plasticiser is propylene glycol.

8. A solution as claimed in any of the preceding Claims wherein the gelling agent is comprised of one or more gums.

9. A solution as claimed in any of the preceding Claims wherein a hydrating agent is included to aid the formation of a solution of the gum.

10. A solution as claimed in Claim 8 wherein the hydrating agent is sodium citrate.

11. A process for producing a solution for sealing capsules comprising the mixing of a first and second subordinate solution, wherein the first solution is aqueous and contains the chemical material of the capsule.

12. A process as claimed in Claim 11 wherein plasticisers are added to the first solution.

13. A process as claimed in Claims 11 or 12 wherein the first solution further includes a solute.

14. A process as claimed in Claims 11-13 wherein the second solution contains a gelling agent and a hydrating agent.

15. A process as claimed in Claims 11-14 wherein the subordinate solutions are heated prior to mixing to form the sealing solution.
